# EUROPEAN PATENT SPECIFICATION

(11) **EP 3 712 615 B1**
(45) Date of publication and mention of the grant of the patent: **04.01.2023**
(21) Application number: 18879821.9
(22) Date of filing: 14.11.2018
(51) Int. Cl.: G01N 33/53, G01N 33/543

(54) **ASSEMBLY-TYPE BLOT STRIP DEVICE**
ANORDNUNGSARTIGE BLOT-STREIFENVORRICHTUNG
DISPOSITIF DE BANDE DE TRANSFERT DE TYPE À ASSEMBLAGE

(30) Priority: 14.11.2017 KR 20170151547
(43) Date of publication of application: 23.09.2020
(73) Proprietor: Sugentech, Inc., Daejeon 34025 (KR)
(72) Inventor: YOO, Seungbum, Daejeon 35263 (KR); KIM, Sungrak, Daejeon 34020 (KR); SOHN, Hosung, Daejeon 34049 (KR)
(74) Representative: Behr, Wolfgang
(86) International application number: PCT/KR2018/013925
(87) International publication number: WO 2019/098680

(56) References cited:
- JP-A- 2008 267 950
- JP-B2- 4 181 499
- KR-A- 20170 088 575
- KR-B1- 101 523 487
- US-B2- 6 514 769
- ERIN M. FENTON ET AL: "Multiplex Lateral-Flow Test Strips Fabricated by Two-Dimensional Shaping", APPLIED MATERIALS & INTERFACES, vol. 1, no. 1, 28 January 2009 (2009-01-28), pages 124-129, XP055410081, US ISSN: 1944-8244, DOI: 10.1021/am800043z
- "SUGENTECH Homepage", Technology Introduction, 22 October 2018 (2018-10-22), pages 1-3, XP009520945, Retrieved from the Internet: URL:http://www.sugentech.com/KOR/technolog y/it.php

## Description

### [Technical Field]

The present invention relates to an assembly-type blot strip device, and more particularly, to an assembly-type blot strip device in which a holder with a blot strip fixed thereonto is mounted in a case in an assembled type to be detachable from the case.

[Background Art] ERIN M. FENTON ET AL: "Multiplex Lateral-Flow Test Strips Fabricated by Two-Dimensional Shaping", APPLIED MATERIALS & INTERFACES, vol. 1, no. 1, 28 January 2009 (2009-01-28), pages 124-129, discloses all types of multi-analyte test devices, but none of the devices discloses a device where the test lines are removably fixed onto the device.

An immunoblot using an antigen-antibody reaction or a Southern blot for gene detection has been widely used as a useful test method for performing multiple diagnoses by detecting an analyte in a sample. Examples of such methods include an allergy immunoblot test, an autoimmune immunoblot test, a human papilloma virus (HPV) genotyping test, and the like.

The blot test may be performed using a blot strip. Here, the blot strip refers to a kind of test paper coated with a reagent containing an antibody that reacts with an antigen, which is an analyte in a sample.

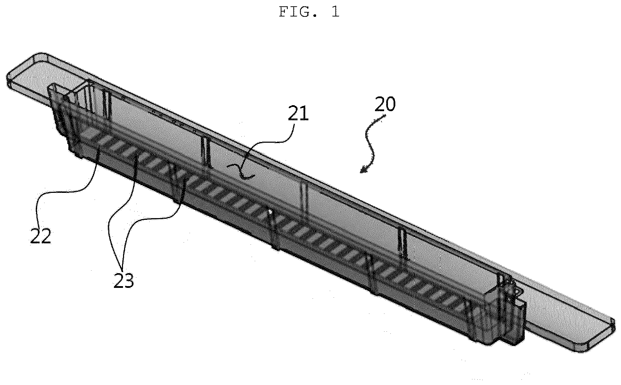

FIG. 1 is an illustrative view schematically illustrating a conventional blot strip device. In a conventional blot strip device 20, one blot strip 22 is fixed onto an inner bottom surface of a case in which a groove 21 is formed, and the blot strip 22 is coated with several to dozens of reagents 23 to perform various kinds of preset tests, thereby performing multiple tests.

The conventional blot strip device 20 is advantageous in that a variety of tests may be simultaneously performed, but has a problem in that unnecessary medical expenses may be incurred by performing a test that is not desired by a user.

### [Disclosure]

### [Technical Problem]

An object of the present invention is to provide an assembly-type blot strip device capable of selectively performing only a test that is desired by a user, without performing preset multiple tests as in a conventional blot strip device.

### [Technical Solution]

In one general aspect, an assembly-type blot strip device includes: a holder including a base and side walls formed in a height direction on both sides of the base to face each other, with a blot strip fixed onto an upper surface of the base; a case having an open upper portion and having a holder groove in which multiple holders are mounted adjacent to each other in a line; and a passage formed by mounting the multiple holders in the holder groove, wherein the holder is mounted in the holder groove in an assembled type to be detachable from the case.

The holder may have one or more assembling protrusions protruding from outer surfaces of the side walls of the holder, and the case may have assembling grooves into which the assembling protrusions are fitted in a vertical direction.

The holder may have multiple assembling protrusions, the multiple assembling protrusions having a trapezoidal shape, a semicircle shape, a tetragonal shape, a triangular shape, or a combination thereof.

A holder index for identifying the holder may be provided on the holder, and a case index identical to the holder index of the holder may be provided on the case.

A fixing means fixing the blot strip may be formed on the upper surface of the base of the holder.

### [Advantageous Effects]

According to the present invention, it is possible to selectively perform only the test that is desired by a user, without performing multiple tests preset as in the conventional blot strip device. Accordingly, an undesired or unnecessary test is not performed, such that test costs are reduced.

### [Description of Drawings]

FIG. 1 is an illustrative view schematically illustrating a conventional blot strip device.
FIG. 2 is a perspective view of an assembly-type blot strip device according to an exemplary embodiment of the present invention.
FIG. 3 is a plan view of FIG. 2 when viewed from above.
FIG. 4 is a perspective view of a holder according to an exemplary embodiment of the present invention.
FIG. 5 is an illustrative view illustrating that a blot strip is fixed onto the holder.
FIG. 6 is a perspective view of a case according to an exemplary embodiment of the present invention.
FIG. 7 is an illustrative view illustrating that the holder is being mounted in the case.
FIG. 8 is an illustrative view illustrating various kinds of holders having assembling protrusions in various shapes.
FIG. 9 is a plan view of an assembly-type blot strip device on which a holder index and a case index are provided, when viewed from above.

### [Description of reference numerals]

10: assembly-type blot strip device
11: passage
100: blot strip 110: blot strip line
200: holder 210: base
220: side wall 230: assembling protrusion
240: holder index 250: fixing means
300: case 310: holder groove
330: assembling groove 340: case index

### [Best Mode]

Hereinafter, an assembly-type blot strip device 10 according to the present invention will be described in detail with reference to the accompanying drawings. The accompanying drawings are provided to sufficiently convey the technical spirit of the present invention to those skilled in the art, and the present invention is not limited only to the accompanying drawings, but may be embodied in another form without changing the technical spirit of the present invention.

Like reference numerals throughout the drawings denote like components, and detailed description of known functions and configurations which may unnecessarily obscure the gist of the present invention will be omitted.

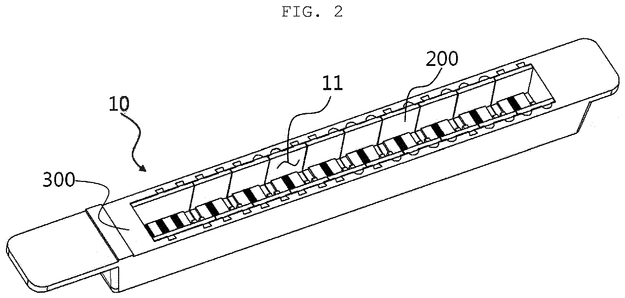

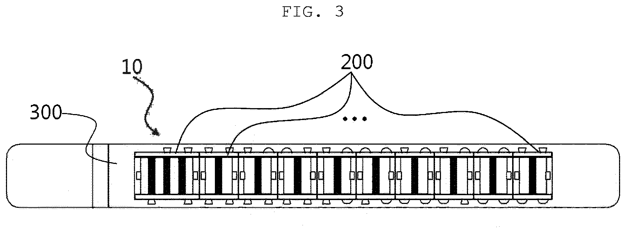

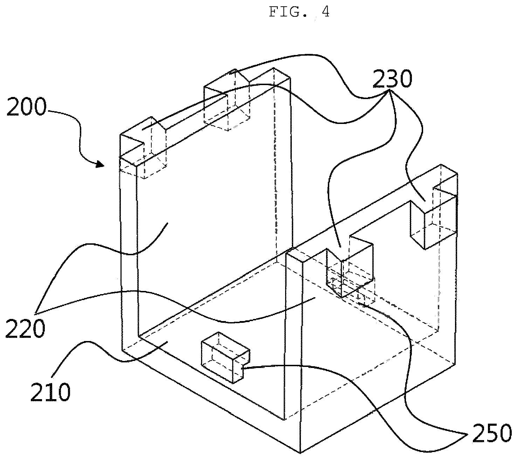

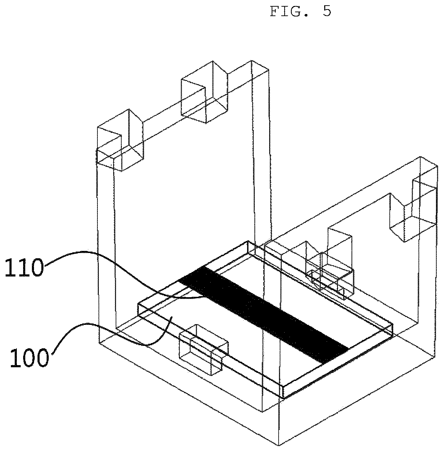

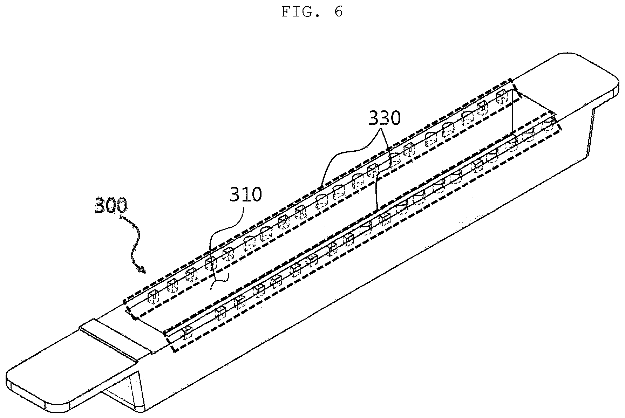

FIG. 2 is a perspective view of an assembly-type blot strip device according to an exemplary embodiment of the present invention, FIG. 3 is a plan view of FIG. 2 when viewed from above, FIG. 4 is a perspective view of a holder according to an exemplary embodiment of the present invention, FIG. 5 is an illustrative view illustrating that a blot strip is fixed onto the holder, and FIG. 6 is a perspective view of a case according to an exemplary embodiment of the present invention.

Referring to FIG. 2, the assembly-type blot strip device 10 according to the present invention mainly includes a holder 200, a case 300, and a passage 11 formed by mounting the holder 200 in the case 300.

Each component will be described in detail. As illustrated in FIG. 4, the holder 200 includes a base 210 and side walls 220 formed in a height direction on both sides of the base 210 to face each other.

A width of the base 210 and a height of the side wall 220 in the holder may correspond to a width and a depth of a holder groove 310 in a case, which will be described below, respectively, so that the holder 200 may be fitted into the holder groove 310, and a length of the base 210 of the holder may be smaller than that of the holder groove 310, so that multiple holders 200 may be mounted in the holder groove 310.

A blot strip 100 is fixed onto an upper surface of the base 210 of the holder as illustrated in FIG. 5. The blot strip 100 may be fixed onto the base 210 of the holder by bonding a lower surface of the blot strip 100 to the upper surface of the base 210 of the holder using a double-sided tape or an adhesive, or may be fixed onto the base 210 of the holder using a fixing means 250 formed on the upper surface of the base 210 of the holder.

To this end, the holder 200 according to an exemplary embodiment of the present invention may further include the fixing means 250 fixing the blot strip 100 onto the upper surface of the base 210 of the holder. Here, the fixing means 250 may be " 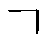 "-shaped hooks protruding upwardly from the upper surface of the base 210 of the holder at both sides thereof as illustrated in FIGS. 4 and 5, and the blot strip 100 is inserted between the hooks and the upper surface of the base 210 of the holder, such that the blot strip 100 may be fixed onto the holder 200. However, the fixing means 250 is not limited to the " 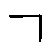 "-shaped hooks.

Meanwhile, a reagent is coated on the blot strip 100, and a blot strip line 110 is thus formed. One or more blot strip lines 110 may be formed in the same size (width) or different sizes (widths).

The case 300 has an open upper portion with the holder groove 310 in which the multiple holders 200 are mounted adjacent to each other in a line, as illustrated in FIG. 6.

The holder groove 310 is formed to be elongated in a longitudinal direction, and is formed to have a length greater than that of the holder 200 (i.e. a length of the base 210 of the holder) to provide a space in which the multiple holders 200 may be mounted in the longitudinal direction.

In the assembly-type blot strip device 10 illustrated in FIG. 2, the number of holders 200 mounted in the case 300 is ten, but the number of holders 200 mounted in the case 300 may be adjusted according to a user's intention. To this end, the case 300 and the holder 200 may be appropriately modified in size.

In addition, the respective holders 210 mounted in the holder groove 310 may be configured to have the same length or different lengths. For example, it may be seen from FIG. 2 that the leftmost holder 210 is formed to have a larger length than the other holders 210, and the other holders 210 are configured to have the same length. However, the lengths of the holders 210 are not limited thereto, and may be modified according to the user's intention.

Meanwhile, as illustrated in FIG. 2, in the assembly-type blot strip device 10 according to the present invention, the passage 11 is formed by mounting the multiple holders 200 in the holder groove 310.

The passage 11 provides a space for a reaction solution containing an antigen to pass between the adjacent holders 200. Accordingly, the reaction solution may pass between all of the holders 200 mounted in the holder groove 310 and be thus in contact with all of the blot strips 100 fixed onto the holders 200.

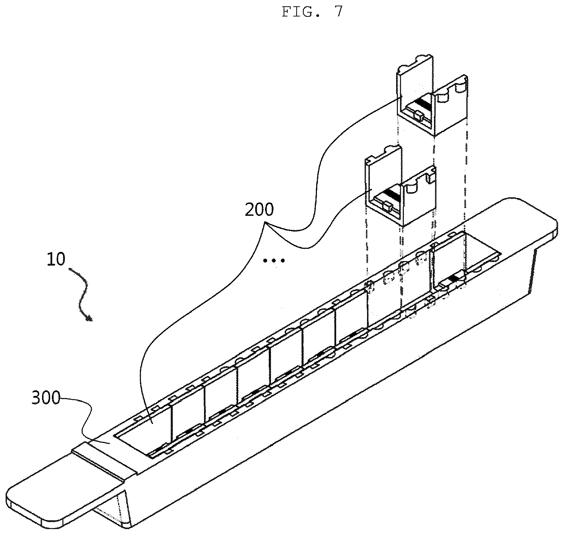

FIG. 7 is an illustrative view illustrating that the holder is mounted in the case 300, and the holder 200 is mounted in the holder groove 310 in an assembled type to be detachable from the case 300.

A user may configure the assembly-type blot strip device 10 to perform only a test desired by the user by fixing the blot strip 100 for performing the test desired by the user onto the holder 200 and mounting the holder 200, onto which the blot strip 100 is fixed, in the holder groove 310 of the case.

That is, the assembly-type blot strip device 10 according to the present invention is capable of selectively performing a test according to a user's selection. Accordingly, an undesired or unnecessary test is not performed, and test costs are thus reduced.

Meanwhile, the holder 200 may have one or more assembling protrusions 230 protruding from outer surfaces of the side walls 220 of the holder as illustrated in FIG. 4, and the case 300 may have assembling grooves 330 into which the assembling protrusions 230 are fitted in a vertical direction as illustrated in FIG. 6.

The assembling protrusions 230 may be formed to protrude outwardly from the outer surfaces of the side walls 220 of the holder 200 and be formed close to upper ends of the side walls 220 of the holder, and the assembling grooves 330 whose number corresponds to the number of holders 200 may be formed in upper ends of inner side walls of the holder groove 310 of the case 300 such that the assembling protrusions 230 of the holder may be fitted thereinto in the vertical direction.

The holder 200 may be mounted in the case 300 in a firmly fixed state by forming the assembling protrusions 230 on the holder 200 and forming the assembling grooves 330 in the case 300 as described above.

In addition, multiple assembling protrusions 230 may be formed on the holder 200. For example, four assembling protrusions 230 are formed on the holder 200 illustrated in FIG. 4, but the number of assembling protrusions 230 is not limited thereto.

In this case, each of the multiple assembling protrusions 230 may have any of various shapes such as a trapezoidal shape, a semicircle shape, a tetragonal shape, and a triangular shape, when viewed from above, and the holder 200 may be configured to have a combination of the assembling protrusions 230 of the various shapes.

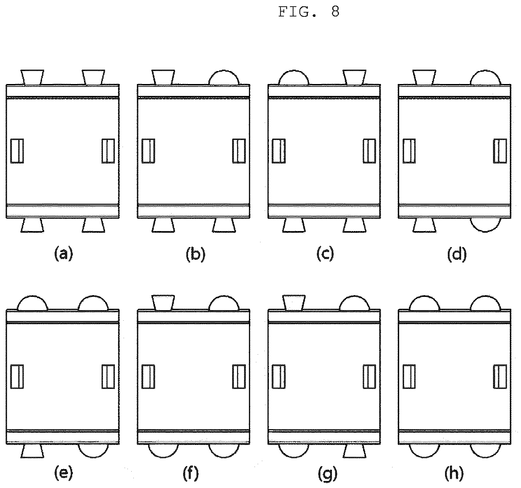

FIG. 8 is an illustrative view illustrating various kinds of holders 200 having the assembling protrusions 230 of various shapes. For example, in FIG. 8A, all of the four assembling protrusions 230 have the trapezoidal shape, and in FIG. 8F, a trapezoidal assembling protrusion 230 and a semicircle assembling protrusion 230 are formed on one of the side walls 220 and only semicircle assembling protrusions 230 are formed on the other one of the side walls 220.

The respective assembling protrusions 230 of the holder may be formed in the same shape or in different shapes, and accordingly, the holder 200 may be configured to have a combination of the multiple assembling protrusions 230 of the various shapes.

That is, each of the holders 200 is configured to have a combination of the assembling protrusions 230 of the various shapes, and the assembling grooves 330 corresponding to the assembling protrusions 230 are formed in the case 300, and thus, the holder 200 may be mounted at a predetermined position of the case 300.

Accordingly, the user may configure the assembly-type blot strip device 10 according to the present invention such that a test desired by the user is performed on the blot strip 100 fixed onto the holder 200 mounted at a position intended by the user. Therefore, in a case of performing a test using the assembly-type blot strip device 10 according to the present invention, it is possible to perform the test desired by the user at the position intended by the user.

In this case, in order to avoid confusion as to a position where the holder 200 is mounted in the case 300, a holder index 240 for identifying the holder 200 may be provided on the holder 200, and a case index 340 identical to the holder index 240 may be provided at a position of the case 300 close to a position where the holder is to be mounted.

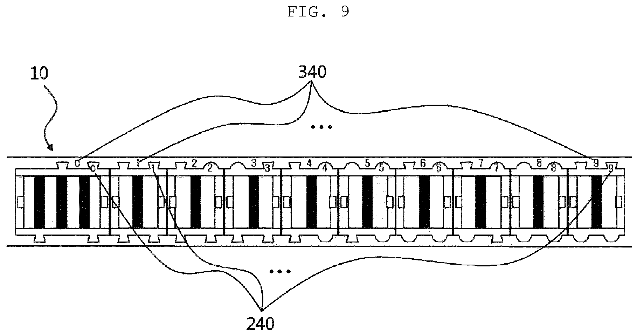

FIG. 9 is a plan view of the assembly-type blot strip device 10 on which the holder index 240 and the case index 340 are provided, when viewed from above. As illustrated in FIG. 9, a unique holder index 240 may be provided on an upper end of the holder 200 to identify each holder 200, and the case index 340 identical to the holder index 240 may be provided on an upper end of the case 300 at a position of the case 300 close to a position where the holder 200 is to be mounted.

For example, in FIG. 9, holder indices 240, such as C, 1, 2, 3, ..., and 9, are provided on the upper end of one of the side walls 220 in the respective holders rightwards from the leftmost holder 200, and case indices 340 identical to the holder indices 240, such as C, 1, 2, 3, ..., and 9, are provided on the upper end of the case 300 at positions close to positions at which the respective holders 200 are to be mounted.

In this case, the holder index 240 and the case index 340 may be numbers, letters, symbols, or the like, and the holder index 240 and the case index 340 are preferably provided at positions well visible to the user. The holder index 240 and the case index 340 may be provided on the upper ends of the holder 200 and the case 300 as described above, but are not limited thereto.

Meanwhile, since the holder index 240 and the case index 340 are provided as described above, it is possible to know, in advance, a position where the holder 200 is to be mounted in the case 300, and it is also possible to determine a forward direction in which the holder 200 is to be mounted in the case 300 on the basis of the forward direction in which the holder index 240 and the case index 340 are provided, such that the user may mount the holder 200 in the case 300 without confusion.

The exemplary embodiment illustrated for describing the present invention is merely one exemplary embodiment in which the present invention is embodied. The scope of protection is defined by the appended claims.

## Claims

1. An assembly-type blot strip device comprising:
a holder including a base and side walls formed in a height direction on both sides of the base to face each other, with a blot strip fixed onto an upper surface of the base;
a case having an open upper portion and having a holder groove in which multiple holders are mounted adjacent to each other in a line; and
a passage formed by mounting the multiple holders in the holder groove,
wherein the holder is mounted in the holder groove in an assembled type to be detachable from the case.

2. The assembly-type blot strip device of claim 1, wherein the holder has one or more assembling protrusions protruding from outer surfaces of the side walls of the holder, and
the case has assembling grooves into which the assembling protrusions are fitted in a vertical direction.

3. The assembly-type blot strip device of claim 2, wherein the holder has multiple assembling protrusions, the multiple assembling protrusions having a trapezoidal shape, a semicircle shape, a tetragonal shape, a triangular shape, or a combination thereof.

4. The assembly-type blot strip device of claim 1, wherein a holder index for identifying the holder is provided on the holder, and a case index identical to the holder index of the holder is provided on the case.

5. The assembly-type blot strip device of claim 1, wherein a fixing means fixing the blot strip is formed on the upper surface of the base of the holder.

## Patentansprüche

1. Blotstreifen-Vorrichtung vom Montagetyp, aufweisend:
einen Halter, der eine Basis und Seitenwände aufweist, die in einer Höhenrichtung auf beiden Seiten der Basis derart ausgebildet sind, dass sie einander zugewandt sind, wobei ein Blotstreifen an einer oberen Oberfläche der Basis befestigt ist;
ein Gehäuse mit einem offenen oberen Abschnitt und mit einer Halternut, in der mehrere Halter nebeneinander in einer Reihe montiert sind; und
einen Durchgang, der durch Anbringen der mehreren Halter in der Halternut gebildet wird,
wobei der Halter in einer montierten Art in der Halternut derart montiert ist, dass er von dem Gehäuse lösbar ist.

2. Blotstreifen-Vorrichtung vom Montagetyp gemäß Anspruch 1, wobei der Halter einen oder mehrere Montagevorsprünge aufweist, die von äußeren Oberflächen der Seitenwände des Halters vorstehen, und
das Gehäuse Montagenuten aufweist, in die die Montagevorsprünge in einer vertikalen Richtung eingesetzt sind.

3. Blotstreifen-Vorrichtung vom Montagetyp gemäß Anspruch 2, wobei der Halter mehrere Montagevorsprünge aufweist, wobei die mehreren Montagevorsprünge eine Trapezform, eine Halbkreisform, eine viereckige Form, eine dreieckige Form oder eine Kombination davon haben.

4. Blotstreifen-Vorrichtung vom Montagetyp gemäß Anspruch 1, wobei ein Halterindex zum Identifizieren des Halters an dem Halter vorgesehen ist, und ein Gehäuseindex, der mit dem Halterindex des Halters identisch ist, an dem Gehäuse vorgesehen ist.

5. Blotstreifen-Vorrichtung vom Montagetyp gemäß Anspruch 1, wobei eine Befestigungseinrichtung, die den Blotstreifen befestigt, an der oberen Oberfläche der Basis des Halters gebildet ist.

## Revendications

1. Dispositif de bande de transfert de type assemblage, comprenant :
un support comportant une base et des parois latérales formées dans un sens de la hauteur sur les deux côtés de la base en se faisant face les unes les autres, avec une bande de transfert fixée sur une surface supérieure de la base ;
un boîtier présentant une partie supérieure ouverte et présentant une rainure de support dans laquelle plusieurs supports sont montés de manière adjacente les uns par rapport aux autres en une ligne ; et
un passage formé en montant les plusieurs supports dans la rainure de support,
dans lequel le support est monté dans la rainure de support selon un type assemblé pour pouvoir être détaché du boîtier.

2. Dispositif de bande de transfert de type assemblage selon la revendication 1, dans lequel le support présente une ou plusieurs parties faisant saillie d'assemblage faisant saillie de surfaces extérieures des parois latérales du support, et
le boîtier présente des rainures d'assemblage, dans lesquelles les parties faisant saillie d'assemblage sont ajustées dans une direction verticale.

3. Dispositif de bande de transfert de type assemblage selon la revendication 2, dans lequel le support présente plusieurs parties faisant saillie d'assemblage, les plusieurs parties faisant saillie d'assemblage présentant une forme trapézoïdale, une forme semi-circulaire, une forme tétragonale, une forme triangulaire ou une combinaison de celles-ci.

4. Dispositif de bande de transfert de type assemblage selon la revendication 1, dans lequel un indice de support d'identification du support est fourni sur le support et un indice de boîtier identique à l'indice de support du support est fourni sur le boîtier.

5. Dispositif de bande de transfert de type assemblage selon la revendication 1, dans lequel un moyen de fixation fixant la bande de transfert est formé sur la surface supérieure sur la base du support.
